# FASCICULE DE BREVET EUROPEEN

(11) **EP 3 188 691 B1**
(45) Date de publication et mention de la délivrance du brevet: **31.10.2018**
(21) Numéro de dépôt: 15766906.0
(22) Date de dépôt: 01.09.2015
(51) Int. Cl.: A61F 2/18

(54) **PROTHESE STAPEDIENNE ET INSTALLATION A COMMANDE MANUELLE OU ROBOTISEE COMPORTANT UNE TELLE PROTHESE**
STEIGBÜGELPROTHESE UND MANUELL GESTEUERTE ODER ROBOTERGESTEUERTE ANLAGE MIT SOLCH EINER PROTHESE
STAPEDIAL PROSTHESIS AND MANUALLY CONTROLLED OR ROBOT-CONTROLLED INSTALLATION COMPRISING SUCH A PROSTHESIS

(30) Priorité: 05.09.2014 FR 1458319
(43) Date de publication de la demande: 12.07.2017
(73) Titulaire: Collin, 92220 Bagneux (FR)
(72) Inventeur: MAZALAIGUE, Stéphane, 94240 L'Haÿ-les-Roses (FR); CZAPLINSKI, Armand, 92220 Bagneux (FR)
(74) Mandataire: Bonnet, Michel
(86) Numéro de dépôt international: PCT/FR2015/052309
(87) Numéro de publication internationale: WO 2016/034806

(56) Documents cités:
- WO-A1-2013/138818
- DE-B3-102009 006 047
- DE-U1-202014 100 093
- US-A1- 2003 130 734
- US-A1- 2008 097 603
- US-A1- 2010 174 292

## Description

La présente invention concerne une prothèse stapédienne et une installation à commande manuelle ou robotisée comportant une telle prothèse.

Elle s'applique plus précisément à une prothèse d'otologie destinée à remplacer un étrier, dernier osselet de la chaîne ossiculaire d'une oreille moyenne d'un patient. A cet effet, elle comporte un corps d'étrier présentant une extrémité proximale pourvue de moyens de fixation à la longue apophyse de l'enclume de l'oreille moyenne du patient et une extrémité distale, prenant généralement la forme d'un cylindre plein et appelée « piston », destinée à être positionnée contre le labyrinthe osseux de l'oreille interne correspondante du patient.

Ce type de prothèse est posé à l'occasion d'une opération chirurgicale de stapédotomie ou stapédectomie au cours de laquelle au moins une partie de l'étrier est extraite de l'oreille moyenne du patient. Plus précisément, lors d'une opération de stapédotomie, un lambeau du tympan est soulevé et l'étrier est partiellement extrait par laser et/ou micro-crochet/ciseau, seule la platine de l'étrier restant en place. Cette platine est alors chirurgicalement perforée à la fraise, au laser ou à la pointe, de manière à pouvoir placer le piston de la prothèse stapédienne dans cette perforation de manière stable. Lors d'une opération de stapédectomie, l'ablation de l'étrier est totale et suivie de son remplacement prosthétique complet. Dans ce cas, la platine de l'étrier peut être remplacée par un greffon d'aponévrose. Dans les deux cas, l'opération est délicate car l'espace de travail est très réduit et l'accès par le conduit auditif externe du patient se fait à l'aide d'un spéculum n'excédant généralement pas 8 mm de diamètre. Elle se fait donc sous microscope et présente des risques d'échec réels, impliquant en outre une sensation d'inconfort permanent du chirurgien pendant l'opération. De plus, les conditions d'accès difficiles et le champ de vision très limité obligent le chirurgien à travailler d'une seule main, l'autre main portant éventuellement une canule d'aspiration et/ou le spéculum. Cette pratique chirurgicale délicate rend l'opération risquée, pourtant très efficace par ailleurs. Cet inconvénient rend en outre problématique la formation des chirurgiens dans ce domaine.

Il s'agit ainsi de l'une des opérations d'otorhinolaryngologie les plus compliquées. Pourtant, cette opération chirurgicale est particulièrement indiquée en cas d'otospongiose, une pathologie liée à une dystrophie osseuse limitée à l'oreille et touchant, dans la grande majorité des cas, l'étrier. Cet osselet est impliqué dans la transmission des ondes sonores et des vibrations du tympan à l'oreille interne et aux cellules sensorielles auditives. L'incidence de l'otospongiose peut être estimée entre 0,1 et 2% d'une population. C'est une maladie évolutive qui peut conduire à des surdités sévères à profondes.

Mais les outils et prothèses conventionnels ne permettent pas de garantir un niveau suffisant de précision. Notamment il est convenu de considérer qu'avec de tels outils, le risque d'échec d'une opération chirurgicale de stapédotomie ou stapédectomie est compris entre 1 et 5% avec dans certains cas rares une perte complète de l'audition ou une paralysie temporaire ou définitive du nerf facial.

Un premier type connu de prothèse stapédienne comporte un corps d'étrier constitué d'un piston et d'une tige dans le prolongement du piston, l'extrémité libre de la tige s'étendant sous la forme d'un crochet ou boucle à extrémité repliée vers l'extérieur. Ce crochet est ainsi apte à entourer la longue apophyse de l'enclume mais doit être positionné et fixé à l'aide d'un outil supplémentaire, par exemple une pince assurant son sertissage. Au cours de cette étape particulièrement délicate de l'opération chirurgicale, le tremblement naturel de la main du chirurgien pose problème et doit être maîtrisé. Une prothèse de ce type est par exemple le K-Piston® à boucle de la société Heinz KURZ GmbH Medizintechnik.

Un autre type connu de prothèse stapédienne comporte un corps d'étrier constitué d'un piston et d'une tige dans le prolongement du piston, l'extrémité libre de la tige étant raccordée à une pince à fonction de clip. Une telle pince peut être considérée comme comportant une mâchoire supérieure conformée de manière à pouvoir prendre appui sur la longue apophyse de l'enclume et une mâchoire inférieure, solidaire de la tige, conformée de manière à pouvoir enserrer la longue apophyse de l'enclume par coopération avec la mâchoire supérieure. Mais pour réduire le risque de nécrose de la longue apophyse, la pince doit avantageusement présenter une forme assez complexe, notamment à bouclage sans contact. Par ailleurs, là encore un outil supplémentaire peut être nécessaire pour aider au positionnement et là encore le tremblement naturel de la main du chirurgien pose problème. Un tir de laser peut même être requis pour les prothèses contenant du nickel titane. Une prothèse de ce type est par exemple le Soft CliP® Piston, le NitiBond® ou le CliP® Piston MVP de la société Heinz KURZ GmbH Medizintechnik.

Un autre type connu de prothèse stapédienne est décrit dans US 2003/130734 A1. Il peut ainsi être souhaité de prévoir une prothèse stapédienne qui permette de s'affranchir d'au moins une partie des problèmes et contraintes précités.

Il est donc proposé une prothèse stapédienne, comportant un corps d'étrier présentant une extrémité proximale pourvue de moyens de fixation à la longue apophyse de l'enclume d'une oreille moyenne d'un patient et une extrémité distale destinée à être positionnée contre le labyrinthe osseux de l'oreille interne correspondante du patient, les moyens de fixation comportant :
- une mâchoire supérieure conformée de manière à pouvoir prendre appui sur la longue apophyse de l'enclume, et
- une mâchoire inférieure conformée de manière à pouvoir enserrer la longue apophyse de l'enclume par coopération avec la mâchoire supérieure,
dans laquelle les moyens de fixation comportent en outre des moyens d'actionnement de l'une des deux mâchoires en rapprochement de l'autre des deux mâchoires, ces moyens d'actionnement étant fixés de façon amovible à l'extrémité proximale du corps d'étrier.

Ainsi, l'intégration de moyens d'actionnement amovibles dans la prothèse stapédienne facilite sa fixation autour de la longue apophyse de l'enclume sans autre outil que la prothèse elle-même et permet le retrait de ces moyens d'actionnement une fois que la prothèse est positionnée et fixée. Accessoirement, ces moyens d'actionnement intégrés et amovibles peuvent éventuellement aider à l'insertion et au positionnement précis de la prothèse. Le geste chirurgical est ainsi facilité de sorte que le risque d'échec est réduit.

De façon optionnelle, les moyens d'actionnement amovibles comportent un bras creux, dont une extrémité distale est fixée de manière amovible à l'extrémité proximale du corps d'étrier, et une tige coulissant à l'intérieur du bras creux, ce coulissement provoquant le rapprochement de ladite une des deux mâchoires vers ladite autre des deux mâchoires.

De façon optionnelle également :
- les mâchoires inférieure et supérieure s'étendent à partir de l'extrémité proximale du corps d'étrier de manière à pouvoir entourer la longue apophyse de l'enclume, et
- les moyens d'actionnement amovibles coopèrent avec la mâchoire inférieure par déformation non élastique de cette dernière pour la rapprocher de la mâchoire supérieure.

De façon optionnelle également, la mâchoire inférieure comporte à son extrémité proximale une encoche d'aide à la déformation.

De façon optionnelle également, les moyens d'actionnement amovibles comportent :
- un bras creux dont une extrémité distale est fixée de manière amovible à la mâchoire supérieure en s'étendant sensiblement dans le prolongement et selon une direction principale du corps d'étrier sans possibilité de rotation relative avec ce dernier, et
- une tige, coulissant à l'intérieur du bras creux, dont une extrémité distale coopère de manière amovible avec la mâchoire inférieure pour l'actionner en rapprochement vers la mâchoire supérieure par coulissement dans le bras creux.

De façon optionnelle également, la fixation amovible du bras creux à la mâchoire supérieure comporte au moins un pion cylindrique, formé sur la surface extérieure du bras creux, coopérant avec au moins un trou formé dans la mâchoire supérieure au voisinage de l'extrémité proximale du corps d'étrier, ledit au moins un pion cylindrique et ledit au moins un trou étant libres en translation relative dans la direction de leurs axes principaux et empêchant toute rotation relative entre le bras creux et la mâchoire supérieure autour de ces axes principaux.

De façon optionnelle également, la coopération amovible de la tige coulissante avec la mâchoire inférieure comporte un ergot formé à une extrémité proximale de la mâchoire inférieure et un renflement cylindrique formé à l'extrémité distale de la tige coulissante, le renflement cylindrique venant en butée contre l'ergot et actionnant la déformation de la mâchoire inférieure par poussée de la tige coulissante dans un sens distal.

De façon optionnelle également, la coopération amovible de la tige coulissante avec la mâchoire inférieure comporte un coin formé à l'extrémité distale de la tige coulissante, le coin venant en butée contre la mâchoire inférieure et actionnant sa déformation par traction de la tige coulissante dans un sens proximal.

Il est également proposé une installation à commande manuelle ou robotisée d'intervention chirurgicale de stapédotomie ou stapédectomie comportant :
- un bras creux déplaçable sur commande manuelle ou robotisée,
- une tige déplaçable en translation à l'intérieur du bras creux sur commande manuelle ou robotisée également,
- une prothèse stapédienne selon l'invention, et
- des moyens de fixation des moyens d'actionnement amovibles de la prothèse stapédienne au bras creux et à la tige de l'installation.

De façon optionnelle, une telle installation à commande manuelle ou robotisée peut comporter :
- une prothèse stapédienne selon l'invention, dont les moyens d'actionnement amovibles comportent un bras creux et une tige coulissante tels que définis précédemment,
- des moyens de fixation d'une extrémité proximale du bras creux des moyens d'actionnement amovibles de la prothèse stapédienne à une extrémité distale du bras creux déplaçable sur commande manuelle ou robotisée de l'installation, assurant ainsi un positionnement manuel ou robotisé de la prothèse stapédienne, et
- des moyens de solidarisation d'une extrémité proximale de la tige coulissante des moyens d'actionnement amovibles de la prothèse stapédienne à une extrémité distale de la tige déplaçable en translation sur commande manuelle ou robotisée de l'installation, assurant ainsi un rapprochement manuel ou robotisé des deux mâchoires.

L'invention sera mieux comprise à l'aide de la description qui va suivre, donnée uniquement à titre d'exemple et faite en se référant aux dessins annexés dans lesquels :
- la figure 1 représente schématiquement en perspective éclatée la structure générale d'une prothèse stapédienne selon un premier mode de réalisation de l'invention,
- la figure 2 représente schématiquement en vue de face assemblée la structure générale de la prothèse stapédienne de la figure 1, selon une première disposition éloignée de ses deux mâchoires,
- la figure 3 représente schématiquement en vue de face assemblée la structure générale de la prothèse stapédienne de la figure 1, selon une deuxième disposition rapprochée de ses deux mâchoires,
- la figure 4 illustre schématiquement une installation robotisée d'intervention chirurgicale de stapédotomie ou stapédectomie incluant la prothèse stapédienne de la figure 1, et
- la figure 5 représente schématiquement en perspective éclatée la structure générale d'une prothèse stapédienne selon un deuxième mode de réalisation de l'invention.

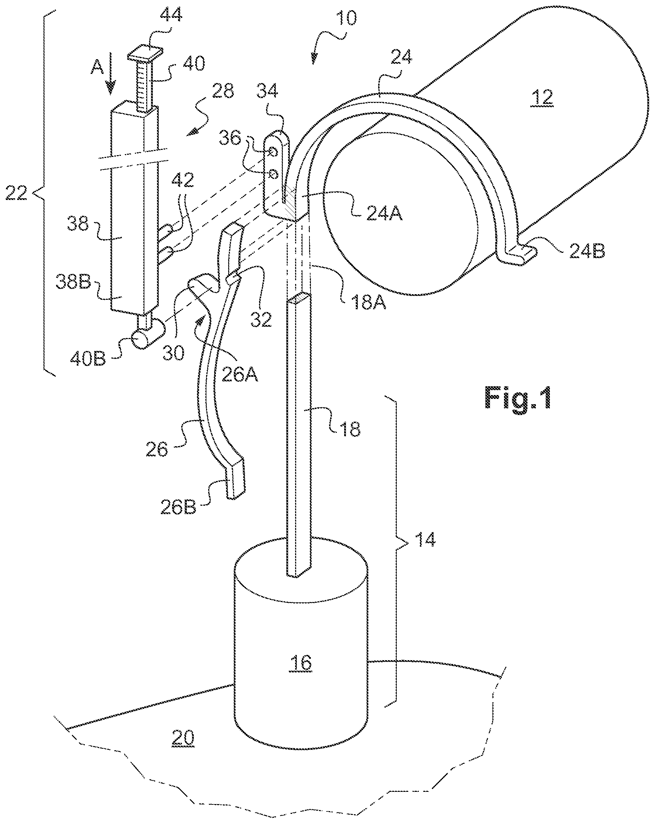

La prothèse stapédienne 10 représentée schématiquement en perspective éclatée sur la figure 1 est destinée à être positionnée et fixée sur la longue apophyse de l'enclume d'une oreille moyenne d'un patient. Une telle longue apophyse est illustrée de façon très schématique et sectionnée par une portion cylindrique 12 sur cette même figure.

La prothèse stapédienne 10 comporte un corps d'étrier 14, ici formé d'un piston 16 et d'une tige 18 fixée au piston dans son prolongement. La base ou extrémité distale du corps d'étrier 14, matérialisée ici par la base du piston 16, est destinée à reposer contre le labyrinthe osseux 20 de l'oreille interne correspondante du patient. L'extrémité proximale du corps d'étrier 14, matérialisée ici par l'extrémité libre 18A de la tige 18 dans la perspective éclatée de la prothèse, est pourvue de moyens 22 de fixation à la longue apophyse 12.

Ces moyens de fixation 22 comportent une mâchoire supérieure 24 recourbée, conformée par exemple en demi-cercle de manière à pouvoir prendre appui sur la longue apophyse 12, et une mâchoire inférieure 26, également recourbée, de manière à pouvoir enserrer de manière sensiblement circulaire la longue apophyse 12 par coopération avec la mâchoire supérieure 24.

Ils comportent en outre des moyens 28 d'actionnement de l'une des deux mâchoires 24 ou 26 en rapprochement de l'autre des deux mâchoires 26 ou 24, ces moyens d'actionnement 28 étant fixés de façon amovible à l'extrémité proximale du corps d'étrier 14. Plus précisément, les moyens d'actionnement 28 envisagés dans l'exemple non limitatif de la figure 1 sont conçus pour rapprocher la mâchoire inférieure 26 de la mâchoire supérieure 24 en étant fixés à l'ensemble solidaire constitué du corps d'étrier 14 et de la mâchoire supérieure 24.

En effet, dans le mode de réalisation non limitatif illustré sur la figure 1, l'extrémité libre 18A de la tige 18 présente une section rectangulaire destinée à être fixée sur une extrémité proximale 24A de même section du demi-cercle que forme la mâchoire supérieure 24. Cette fixation peut se faire, selon le matériau employé pour former la mâchoire supérieure 24 et la tige 18, par collage, soudure, ou même par moulage de la mâchoire supérieure 24 et de la tige 18 en un même dispositif matériel. Elle est illustrée sur la figure 1 par la section hachurée de l'extrémité libre 18A de la tige 18 et par des pointillés reliant cette section hachurée à l'extrémité proximale 24A de la mâchoire supérieure 24.

Dans le mode de réalisation de la figure 1 également, les mâchoires supérieure 24 et inférieure 26 sont destinées à être fixées latéralement l'une contre l'autre. Par exemple, une extrémité proximale 26A du demi-cercle que forme la mâchoire inférieure 26 est latéralement fixée à l'extrémité proximale 24A de la mâchoire supérieure 24. Cette fixation est illustrée sur la figure 1 par une zone hachurée délimitée latéralement à l'extrémité proximale 24A de la mâchoire supérieure 24 et par des pointillés reliant latéralement cette zone hachurée à une partie supérieure de l'extrémité proximale 26A de la mâchoire inférieure 26. Elle peut se faire, selon le matériau employé pour former la mâchoire supérieure 24 et la mâchoire inférieure 26, par collage, point de soudure, ou même éventuellement par moulage de la mâchoire supérieure 24, de la tige 18 et de la mâchoire inférieure 26 en un même dispositif matériel si la conformation générale de la prothèse le permet. Il est à noter cependant qu'il peut y avoir un avantage industriel à fabriquer indépendamment les mâchoires inférieure et supérieure, ce qui ajoute à l'intérêt de les fixer soit par collage soit à l'aide d'un point de soudure.

L'extrémité distale 24B du demi-cercle que forme la mâchoire supérieure 24 peut être repliée vers l'extérieur en un léger crochet pour faciliter le positionnement sans dommage de cette mâchoire supérieure 24 sur la longue apophyse 12.

Par ailleurs, un ergot 30 est prévu à l'extrémité proximale 26A de la mâchoire inférieure 26 et s'étend vers l'extérieur de la courbure qu'elle forme. Cet ergot 30 est néanmoins situé sous la partie supérieure de l'extrémité proximale 26A de la mâchoire inférieure 26 qui est fixée latéralement à l'extrémité proximale 24A de la mâchoire supérieure 24. Il est destiné à coopérer avec les moyens d'actionnement 28 pour diriger l'extrémité distale 26B de la mâchoire inférieure 26 vers l'extrémité distale 24B de la mâchoire supérieure 24, par déformation non élastique de la mâchoire inférieure 26. Cette déformation a lieu selon un axe situé entre la zone de fixation de l'extrémité proximale 26A de la mâchoire inférieure 26 et l'ergot 30, en appuyant sur ce dernier vers le bas. Elle peut être facilitée par la présence d'une encoche 32 aménagée dans la mâchoire inférieure 26 en cet endroit. On notera aussi que la mâchoire inférieure 26 peut présenter une certaine déformation latérale, de sorte que bien que fixée latéralement à la mâchoire supérieure 24, leurs extrémités distales 24B et 26B restent disposées en regard l'une de l'autre.

Par ailleurs également, une extension 34 est formée dans la mâchoire supérieure 24 à son extrémité proximale 24A et s'étend vers le haut au-dessus de l'ergot 30. Elle comporte deux trous 36 destinés à coopérer avec les moyens d'actionnement 28 pour fixer ces derniers de façon amovible à la mâchoire supérieure 24, d'une façon qui va maintenant être détaillée.

Les moyens d'actionnement 28 comportent en effet :
- un bras creux 38, présentant un conduit cylindrique (de section circulaire ou autre), dont une extrémité distale 38B est fixée de manière amovible à la mâchoire supérieure 24 en s'étendant par exemple sensiblement dans le prolongement du corps d'étrier 14, et
- une tige cylindrique 40 (de section circulaire ou autre), coulissant à l'intérieur du bras creux 38 dans son conduit cylindrique, dont une extrémité distale 40B coopère de manière amovible avec la mâchoire inférieure 26 pour l'actionner en la rapprochant de la mâchoire supérieure 24 par coulissement dans le bras creux 38.

Plus précisément, le bras creux 38 comporte à son extrémité distale 38B deux pions cylindriques 42 s'étendant en saillie dans la direction des deux trous 36 formés dans la mâchoire supérieure 24, cette direction étant parallèle à celle de l'axe de déformation de la mâchoire inférieure 26. Les pions cylindriques 42 sont dimensionnés pour s'insérer et sortir aisément des trous 36 tout en assurant la fixation amovible du bras creux 38 à la mâchoire supérieure 24. Grâce aux deux pions cylindriques 42 et aux deux trous 36, la fixation interdit toute rotation relative entre les moyens d'actionnement 28 et le corps de l'étrier 14, ce qui assure un bon maintien sans basculement de la prothèse stapédienne 10 lors de son positionnement et de sa fixation. Le seul degré de liberté permis dans cette fixation est la translation selon l'axe des pions cylindriques 42 et des trous 36 qui permet le retrait latéral des moyens d'actionnement 28 une fois que la prothèse stapédienne 10 est posée et fixée autour de la longue apophyse 12. Deux trous 36 sont représentés avec des sections circulaires dans la figure 1 mais peuvent en variante être remplacés par un seul trou de section oblongue (coopérant alors avec un seul pion cylindrique de section oblongue) pour faciliter ce retrait latéral avec la même contrainte d'empêcher tout basculement de la prothèse. On notera aussi que le bras creux 38 est illustré très schématiquement de forme parallélépipédique rectangle mais peut présenter toute autre forme adaptée et aisément fabricable telle qu'une forme cylindrique circulaire par exemple. Enfin, on notera que le dégagement nécessaire latéralement pour retirer les moyens d'actionnement 28 après la pose de la prothèse stapédienne 10 ne présente pas un encombrement particulièrement gênant à partir du moment où il reste inférieur au rayon du piston 16 ou à une fois et demie l'épaisseur de l'extension 34.

On notera par ailleurs que les éléments constitutifs de la prothèse stapédienne 10 sont de petite taille, celle-ci ne dépassant pas elle-même quelques millimètres. Ainsi, en complément ou en remplacement des moyens de fixation amovible à trou(s) et pion(s) coopérant entre eux, il est possible de prévoir un dispositif de fixation amovible par aspiration/surpression comme cela est bien connu en microchirurgie.

Plus précisément également, l'extrémité distale 40B de la tige coulissante 40 s'étend au-delà de l'extrémité distale 38B du bras creux 38 dans lequel elle coulisse et se présente sous la forme d'un renflement cylindrique d'axe principal parallèle à la direction des trous 36 et des pions cylindriques 42. Ce renflement cylindrique 40B vient en butée contre l'ergot 30 au-dessus de ce dernier et est libre en translation relative avec lui selon cet axe principal pour faciliter l'extraction latérale des moyens d'actionnement 28. Ainsi, tout coulissement de la tige 40 dans le bras creux 38 vers le bas, comme indiqué par la flèche A, permet, par coopération du renflement cylindrique 40B et de l'ergot 30, de pousser ce dernier vers le bas, ce qui a pour effet de déformer la mâchoire inférieure 26 de manière à rapprocher son extrémité distale 26B de l'extrémité distale 24B de la mâchoire supérieure 24 et à réduire le diamètre d'enserrement des deux mâchoires. En partie proximale s'étendant hors du bras creux 38, la tige coulissante 40 peut présenter une graduation ou un curseur permettant à l'utilisateur de visualiser dans quelle mesure l'ergot 30 a été poussé par la tige coulissante 40 et donc dans quelle mesure le diamètre d'enserrement de la longue apophyse 12 a été réduit. Enfin, conformément à une utilisation directe manuelle possible des moyens d'actionnement 28, des moyens 44 de préhension des moyens d'actionnement 28 et de pression sur la tige coulissante 40 peuvent être prévus.

Tous les éléments constitutifs de la prothèse stapédienne 10 décrits précédemment peuvent être conçus en acier inoxydable, titane ou en d'autres matériaux adaptés à une application chirurgicale en otorhinolaryngologie. En ce qui concerne la mâchoire inférieure 26, elle doit en outre être conçue dans un matériau déformable et sans mémoire de forme, comme par exemple le titane. En ce qui concerne l'extrémité distale 40B de la tige coulissante 40, elle peut en outre avantageusement présenter un coefficient de friction aussi faible que possible pour coopérer efficacement avec l'ergot 30, une matière lissée respectant cette propriété.

On notera également que la partie amovible de la prothèse stapédienne 10, à savoir le bras creux 38 et la tige coulissante 40 des moyens d'actionnement 28, peut être fabriquée de manière standard, alors que le corps d'étrier 14 et les mâchoires 24 et 26 peuvent être fabriqués sur mesure ou selon différentes dimensions prédéterminées pour pouvoir s'adapter à une pluralité de morphologies possibles des patients. En particulier, l'ergot 30 peut être de formes et dimensions variables pour permettre des rapprochements maximaux entre les deux mâchoires différents d'un patient à l'autre et précisément définis de manière à éviter tout risque de nécrose par compression de la longue apophyse 12. En particulier également, la prothèse stapédienne 10 peut être fabriquée selon différentes tailles, de telle sorte que la distance entre la base du corps d'étrier 14 et l'axe de la longue apophyse 12 en position fixée de la prothèse soit comprise entre 4 et 6 mm, ou plus précisément entre 4,2 et 4,8 mm.

Enfin, de nombreuses variantes peuvent être apportées au mode de réalisation de la figure 1 dans lequel la tige 18 du corps d'étrier 14 et les mâchoires 24 et 26 sont représentées sous la forme de lames de section rectangulaire, la lame formant la tige 18 s'étendant dans le prolongement de l'extrémité proximale 24A de la lame formant la mâchoire supérieure 24. En particulier, en variante, la tige 18 est conçue pour garder sa stabilité mais n'est pas nécessairement conçue sous forme de lame. En variante également, la mâchoire supérieure 24 pourrait être plus courte, sans extrémité distale 24B repliée et en forme de gouttière pour accueillir l'extrémité distale 26B de la mâchoire inférieure 26, cette mâchoire inférieure 26 étant alors plus longue pour être plus facilement visible lors du positionnement et de la fixation de la prothèse.

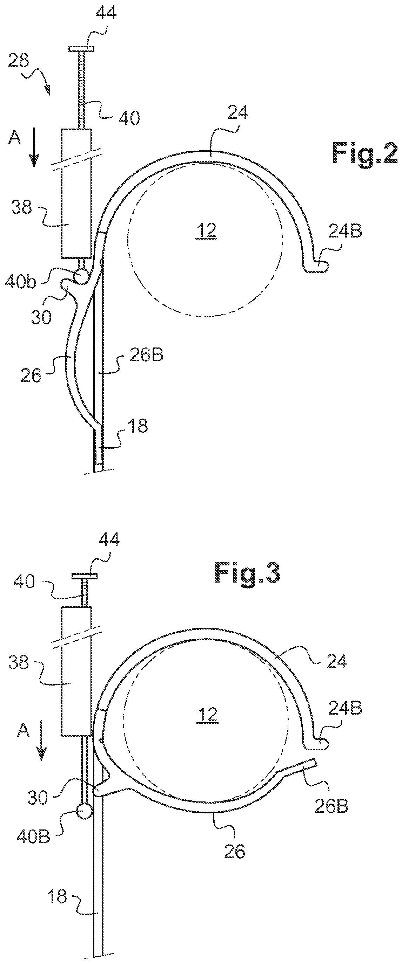

La prothèse stapédienne 10 est représentée en vue de face assemblée sur la figure 2 selon une première disposition éloignée de ses deux mâchoires 24 et 26. C'est précisément la disposition dans laquelle elle doit se présenter avant sa pose et sa fixation autour de la longue apophyse 12. Dans cette disposition, la tige coulissante 40 est rétractée dans le bras creux 38 des moyens d'actionnement 28 et bute contre l'ergot 30 de la mâchoire inférieure 26 sans pour autant exercer de pression particulière contre lui. La mâchoire inférieure 26, qui n'est alors pas déformée, est très ouverte de manière à faciliter le positionnement des mâchoires de la prothèse stapédienne 10 autour de la longue apophyse 12 de l'enclume. Son extrémité distale 26B est par exemple rétractée jusqu'au niveau de la tige 18.

Il convient de noter que les moyens d'actionnement 28, fixés de façon amovible à la mâchoire supérieure 24 grâce à l'engagement des pions cylindriques 42 dans les trous 36, sont disposés sensiblement dans le prolongement et selon une direction principale du reste de la prothèse, notamment du corps d'étrier 14. Dans la perspective illustrée par la figure 2, cette direction principale est la direction verticale. C'est justement la direction dans laquelle la prothèse stapédienne 10 doit être introduite dans l'oreille moyenne du patient. Les moyens d'actionnement 28 remplissent donc non seulement une fonction de rapprochement des deux mâchoires 24, 26 mais également une fonction d'aide à l'introduction et au positionnement de la prothèse stapédienne 10 dans l'oreille moyenne.

La prothèse stapédienne 10 est représentée en vue de face assemblée sur la figure 3 selon une deuxième disposition de rapprochement maximal de ses deux mâchoires 24 et 26, c'est-à-dire de diamètre d'enserrement minimal. Cette disposition est obtenue après déplacement vers le bas, i.e. dans le sens de la flèche A, de la tige coulissante 40. En opérant ce déplacement, le renflement cylindrique distal 40B de la tige coulissante 40 pousse l'ergot 30 vers le bas, ce qui tend à déformer la mâchoire inférieure 26 autour de son axe de déformation et à rapprocher progressivement son extrémité distale 26B de celle de la mâchoire supérieure 24.

Mais en étant poussé vers le bas, l'ergot 30 accompagne la déformation de la mâchoire inférieure 26 et est également progressivement escamoté par rotation autour de l'axe de déformation. De la sorte, il arrive un moment où la butée du renflement cylindrique distal 40B contre l'ergot 30 ne se fait plus, ce qui correspond à la déformation maximale de la mâchoire inférieure 26 illustrée sur la figure 3. Cela correspond également au diamètre d'enserrement minimal souhaité autour de la longue apophyse 12, de sorte que la prothèse stapédienne 10 peut être considérée comme correctement fixée et que les moyens d'actionnement 28 peuvent alors être retirés latéralement selon la direction principale commune des pions cylindriques 42, des trous 36 et du renflement cylindrique distal 40B.

Il convient de noter que la fixation de la prothèse stapédienne 10 autour de la longue apophyse 12 se fait par poussée vers le bas, i.e. selon le sens indiqué par la flèche A, de la tige coulissante 40 dans le bras creux 38. Ce geste se fait donc également dans le prolongement et selon la direction principale de la prothèse stapédienne 10 ce qui facilite l'opération chirurgicale qui peut être réalisée en un seul geste linéaire. Cette opération facilitée est également plus précise. Notamment, lorsque la tige coulissante 40 comporte une graduation ou un curseur, l'enserrement de la longue apophyse 12 par les deux mâchoires 24 et 26 peut être précisément réglé sans risque de nécrose par compression.

Il convient également de noter que la direction de retrait des moyens d'actionnement amovibles 28 est judicieusement orthogonale à celle de l'opération chirurgicale de positionnement et fixation, ce qui limite les risques d'une mauvaise manipulation.

Enfin, le positionnement et la fixation de la prothèse stapédienne 10 se faisant avantageusement dans sa direction principale, cela rend en outre cette prothèse aisément compatible avec une installation robotisée d'intervention chirurgicale de stapédotomie ou stapédectomie.

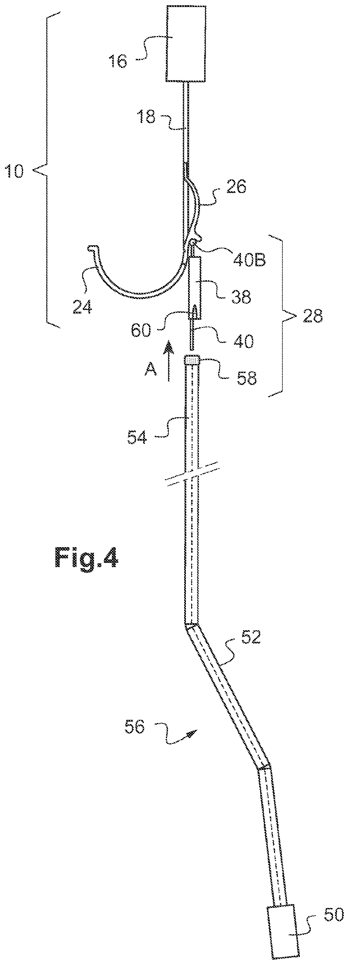

Une telle installation est illustrée de façon très schématique sur la figure 4. Elle comporte un robot 50 muni d'un bras creux 52 déplaçable sur commande électronique et d'une tige 54 déplaçable en translation par coulissement à l'intérieur du bras creux 52 sur commande électronique également. Le bras 52 du robot 50 est déplaçable notamment en translation dans la direction du positionnement et de la fixation de la prothèse stapédienne 10. Il présente une double coudée 56 permettant de déporter l'axe d'intervention pour dégager l'espace opératoire visible par le chirurgien. Un exemple de robot compatible avec une installation selon l'invention est donné dans l'article de Miroir et al, intitulé « RobOtol: from design to évaluation of a robot for middle ear surgery », publié à l'occasion de la conférence IROS 2010 (International Conférence on Intelligent RObots and Systems) qui s'est tenue du 18 au 22 octobre 2010 à Taipei (TW).

L'installation illustrée sur la figure 4 comporte en outre :
- la prothèse stapédienne 10 dans sa première disposition éloignée des mâchoires 24 et 26 (c'est-à-dire avant positionnement et fixation), et
- des moyens de fixation des moyens d'actionnement amovibles 28 de la prothèse stapédienne 10 au bras creux 52 et à la tige 54 du robot 50.

Ces moyens de fixation comportent par exemple une bague de serrage 58 disposée à l'extrémité distale libre du bras creux 52 du robot 50, ce bras creux 52 étant lui-même destiné à recevoir par insertion l'extrémité proximale du bras creux 38 des moyens d'actionnement 28 de la prothèse stapédienne 10. Une encoche longitudinale 60 peut être prévue dans la surface externe du bras creux 38 des moyens d'actionnement 28 de manière à accompagner et guider angulairement son insertion dans le bras creux 52 du robot 50.

Tous moyens de fixation connus et compatibles avec la configuration de l'installation illustrée sur la figure 4 sont envisageables, notamment tous moyens permettant de fixer la prothèse stapédienne 10 de telle sorte que son axe principal corresponde avec l'axe déporté d'intervention du robot 50. Par ailleurs, le système de verrouillage défini dans la demande de brevet français publiée sous le numéro FR 2 998 344 A1 est adapté pour réaliser une telle fixation du bras creux 38 des moyens d'actionnement 28 au bras creux 52 du robot 50.

Il convient de noter qu'une fixation spécifique de la tige coulissante 40 des moyens d'actionnement 28 à la tige 54 du robot 50 est possible mais pas indispensable dans le mode de réalisation de la prothèse stapédienne 10 telle qu'illustrée dans les figures 1 à 3. En effet, le rapprochement des deux mâchoires 24 et 26 étant assuré en poussant la tige coulissante 40 à l'intérieur du bras creux 38 dans le sens distal (sens indiqué par la flèche A), il suffit que l'extrémité distale de la tige 54 du robot 50 soit en contact avec l'extrémité proximale de la tige coulissante 40 pour que cette poussée puisse être transmise de la tige 54 du robot 50 à la tige coulissante 40 des moyens d'actionnement 28.

On notera également qu'en variante, le robot 50 peut être remplacé par une extrémité manipulable manuellement, comportant par exemple des moyens de préhension classiques et des moyens d'actionnement manuel ou semi-manuel de la translation de la tige 54 à l'intérieur du bras creux 52, par exemple à l'aide d'une gâchette, d'un poussoir, ou de tout autre moyen mécanique ou électromécanique équivalent connu.

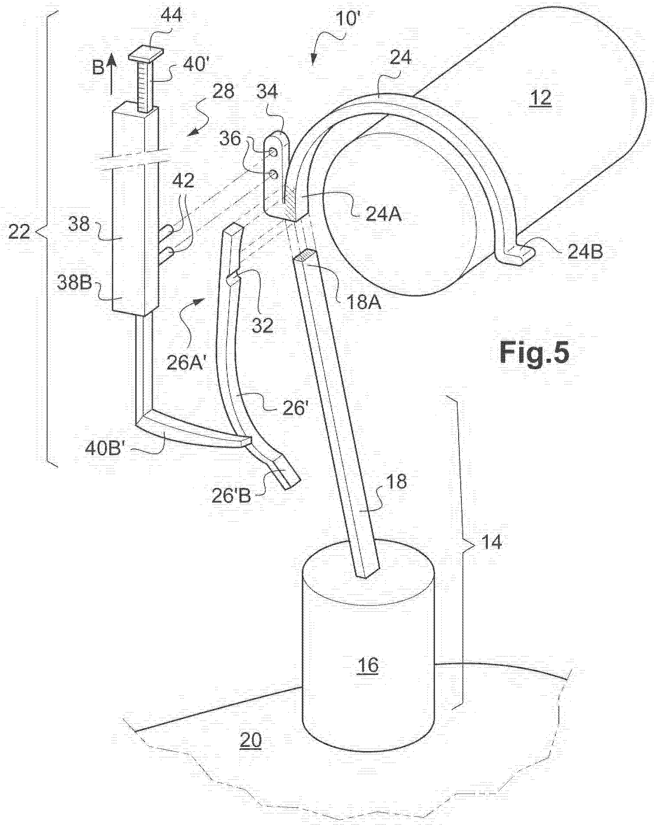

La figure 5 illustre un deuxième mode de réalisation possible de prothèse stapédienne conforme à l'invention, selon une perspective éclatée comparable à celle de la figure 1. La prothèse stapédienne 10' représentée schématiquement sur cette figure est identique à la prothèse stapédienne 10 à l'exception des éléments suivants :
- la mâchoire inférieure 26 est remplacée par une mâchoire inférieure 26' dans laquelle l'ergot 30 est supprimé,
- la tige coulissante 40 est remplacée par une tige coulissante 40' dans laquelle l'extrémité distale 40B en forme de renflement cylindrique est remplacée par une extrémité distale 40B' en forme de coin, et
- la tige 18 est fixée au piston 16 sensiblement dans son prolongement avec cependant une légère inclinaison de la tige 18 par rapport à l'axe principal du piston 16 pour une question d'équilibre et de stabilité de la prothèse dans son ensemble : notamment pour que le piston 16 se trouve juste en dessous de la longue apophyse 12.

Les autres éléments constitutifs de la prothèse stapédienne 10' sont identiques à ceux de la prothèse stapédienne 10 et portent les mêmes références.

Conformément à ce deuxième mode de réalisation, c'est en tirant la tige coulissante 40' vers le haut dans le sens indiqué par la flèche B, i.e. dans le sens inverse de la flèche A, que l'on rapproche les deux mâchoires 24 et 26'. En effet, dans une première disposition étendue de la tige coulissante 40' dans le bras creux 38 des moyens d'actionnement 28, l'extrémité distale 40B' en forme de coin s'étend au-delà de la mâchoire inférieure 26'. Mais en tirant sur la tige coulissante 40' vers le haut, la surface supérieure de l'extrémité distale 40B' en forme de coin vient buter contre la mâchoire inférieure 26' et la déforme vers le haut selon le même principe que celui décrit précédemment, pour rapprocher progressivement son extrémité distale 26B' de celle de la mâchoire supérieure 24.

On notera que dans ce mode de réalisation, le geste chirurgical de pose et de fixation de la prothèse stapédienne 10' est le même que dans le premier mode de réalisation, à l'exception du fait que c'est en tirant sur la tige coulissante 40' des moyens d'actionnement 28 dans la direction principale de la prothèse, et non pas en poussant, que l'on enserre la longue apophyse 12 entre les deux mâchoires 24 et 26'.

Ce mode de réalisation est par conséquent lui aussi tout à fait compatible avec l'insertion de la prothèse stapédienne 10' dans une installation robotisée (ou manuelle) telle que celle de la figure 4. Dans ce cas cependant, il est nécessaire de prévoir des moyens de fixation spécifiques de l'extrémité proximale de la tige coulissante 40' des moyens d'actionnement 28 à l'extrémité distale de la tige 54 du robot 50. En effet, le rapprochement des deux mâchoires 24 et 26' étant assuré en tirant sur la tige coulissante 40' à l'intérieur du bras creux 38 dans le sens proximal (sens indiqué par la flèche B), un simple contact entre l'extrémité distale de la tige 54 du robot 50 et l'extrémité proximale de la tige coulissante 40' n'est pas suffisant. Mais une telle fixation spécifique étant par ailleurs tout à fait connue en soi, elle ne sera pas détaillée.

Il apparaît clairement qu'une prothèse stapédienne telle que l'une de celles décrites précédemment permet de faciliter le geste chirurgical au cours d'une opération de stapédotomie ou stapédectomie, qu'elle soit utilisée manuellement ou intégrée dans une installation robotisée. L'objectif d'une telle prothèse à l'ergonomie améliorée, éventuellement associée à une installation robotisée d'assistance à l'acte médical, est de rendre l'intervention du chirurgien plus sûre, plus précise, moins invasive et plus efficace. Elle permet d'envisager une augmentation des cas pathologiques où une opération chirurgicale pourra avantageusement être conseillée sans risque sensible d'aggraver l'état du patient.

De plus, le fait que les moyens d'actionnement soient amovibles rend la fabrication et la commercialisation de la prothèse plus aisées. En effet, il est possible de produire et distribuer les moyens d'actionnement avec ou sans le reste de la prothèse. En particulier, il est possible de produire des moyens d'actionnement standard, comportant par exemple des moyens de fixation standard à des installations robotisées d'intervention chirurgicale ou adaptés à une utilisation manuelle, alors que le reste de la prothèse peut être produit sur mesure pour chaque patient ou avec des formes et dimensions diverses prédéterminées et indépendamment de l'intervention manuelle ou robotisée qui les utilisera. Ainsi, les moyens d'actionnement amovibles et le reste de la prothèse peuvent être conditionnés et fournis ensemble ou séparément. Ils peuvent également être conçus pour un usage unique en étant conditionnés après stérilisation.

On notera par ailleurs que l'invention n'est pas limitée aux modes de réalisation décrits précédemment.

En particulier, bien que les modes de réalisation décrits précédemment montrent des prothèses stapédiennes dans lesquelles les moyens d'actionnement agissent sur la mâchoire inférieure pour rapprocher son extrémité distale de celle de la mâchoire supérieure, ces moyens d'actionnement pourraient en variante agir sur la mâchoire supérieure qui serait alors mobile ou déformable.

En particulier également, la forme des différents éléments constitutifs de la prothèse peut varier selon les enseignements bien connus par ailleurs dans le domaine de la stapédotomie ou stapédectomie. La tige 18 a par exemple été illustrée et présentée droite, dans le prolongement du piston 16 ou légèrement inclinée. En variante, elle peut présenter une ou plusieurs courbures plus ou moins marquées, notamment selon une forme de baïonnette, pour placer le piston 16 en dessous de la longue apophyse 12. De même, les mâchoires supérieure et inférieure peuvent présenter des bouclages pour réduire les risques de nécrose de la longue apophyse.

Il apparaîtra plus généralement à l'homme de l'art que diverses modifications peuvent être apportées aux modes de réalisation décrits ci-dessus, à la lumière de l'enseignement qui vient de lui être divulgué. Dans les revendications qui suivent, les termes utilisés ne doivent pas être interprétés comme limitant les revendications aux modes de réalisation exposés dans la présente description, mais doivent être interprétés pour y inclure tous les équivalents que les revendications visent à couvrir du fait de leur formulation et dont la prévision est à la portée de l'homme de l'art en appliquant ses connaissances générales à la mise en oeuvre de l'enseignement qui vient de lui être divulgué.

## Revendications

1. Prothèse stapédienne (10; 10'), comportant un corps d'étrier (14) présentant une extrémité proximale pourvue de moyens (22) de fixation à la longue apophyse (12) de l'enclume d'une oreille moyenne d'un patient et une extrémité distale destinée à être positionnée contre le labyrinthe osseux (20) de l'oreille interne correspondante du patient, les moyens de fixation (22) comportant :
- une mâchoire supérieure (24) conformée de manière à pouvoir prendre appui sur la longue apophyse (12) de l'enclume,
- une mâchoire inférieure (26 ; 26') conformée de manière à pouvoir enserrer la longue apophyse (12) de l'enclume par coopération avec la mâchoire supérieure (24), et
- des moyens (28) d'actionnement de l'une des deux mâchoires (26, 24 ; 26', 24) en rapprochement de l'autre des deux mâchoires (24, 26 ; 24, 26'), ces moyens d'actionnement (28) étant fixés de façon amovible à l'extrémité proximale du corps d'étrier (14),
**caractérisée en ce que** les moyens d'actionnement amovibles (28) comportent un bras creux (38), dont une extrémité distale (38B) est fixée de manière amovible à l'extrémité proximale du corps d'étrier (14), et une tige (40 ; 40') coulissant à l'intérieur du bras creux (38), ce coulissement provoquant le rapprochement de ladite une des deux mâchoires (26, 24 ; 26', 24) vers ladite autre des deux mâchoires (24, 26 ; 24, 26').

2. Prothèse stapédienne (10 ; 10') selon la revendication 1, dans laquelle :
- les mâchoires inférieure (26 ; 26') et supérieure (24) s'étendent à partir de l'extrémité proximale du corps d'étrier (14) de manière à pouvoir entourer la longue apophyse (12) de l'enclume, et
- les moyens d'actionnement amovibles (28) coopèrent avec la mâchoire inférieure (26 ; 26') par déformation non élastique de cette dernière pour la rapprocher de la mâchoire supérieure (24).

3. Prothèse stapédienne (10 ; 10') selon la revendication 2, dans laquelle la mâchoire inférieure (26 ; 26') comporte à son extrémité proximale (26A ; 26A') une encoche (32) d'aide à la déformation.

4. Prothèse stapédienne (10 ; 10') selon la revendication 2 ou 3, dans laquelle les moyens d'actionnement amovibles (28) comportent :
- un bras creux (38) dont une extrémité distale (38B) est fixée de manière amovible à la mâchoire supérieure (24) en s'étendant sensiblement dans le prolongement et selon une direction principale du corps d'étrier (14) sans possibilité de rotation relative avec ce dernier, et
- une tige (40 ; 40'), coulissant à l'intérieur du bras creux (38), dont une extrémité distale (40B ; 40B') coopère de manière amovible avec la mâchoire inférieure (26 ; 26') pour l'actionner en rapprochement vers la mâchoire supérieure (24) par coulissement dans le bras creux (38).

5. Prothèse stapédienne (10 ; 10') selon la revendication 4, dans laquelle la fixation amovible du bras creux (38) à la mâchoire supérieure (24) comporte au moins un pion cylindrique (42), formé sur la surface extérieure du bras creux (38), coopérant avec au moins un trou (36) formé dans la mâchoire supérieure (24) au voisinage de l'extrémité proximale du corps d'étrier (14), ledit au moins un pion cylindrique (42) et ledit au moins un trou (36) étant libres en translation relative dans la direction de leurs axes principaux et empêchant toute rotation relative entre le bras creux (38) et la mâchoire supérieure (24) autour de ces axes principaux.

6. Prothèse stapédienne (10) selon la revendication 4 ou 5, dans laquelle la coopération amovible de la tige coulissante (40) avec la mâchoire inférieure (26) comporte un ergot (30) formé à une extrémité proximale (26A) de la mâchoire inférieure (26) et un renflement cylindrique (40B) formé à l'extrémité distale de la tige coulissante (40), le renflement cylindrique (40B) venant en butée contre l'ergot (30) et actionnant la déformation de la mâchoire inférieure (26) par poussée de la tige coulissante (40) dans un sens distal (A).

7. Prothèse stapédienne (10') selon la revendication 4 ou 5, dans laquelle la coopération amovible de la tige coulissante (40') avec la mâchoire inférieure (26') comporte un coin (40B') formé à l'extrémité distale de la tige coulissante (40'), le coin (40B') venant en butée contre la mâchoire inférieure (26') et actionnant sa déformation par traction de la tige coulissante (40') dans un sens proximal (B).

8. Installation à commande manuelle ou robotisée d'intervention chirurgicale de stapédotomie ou stapédectomie comportant :
- un bras creux (52) déplaçable sur commande manuelle ou robotisée,
- une tige (54) déplaçable en translation à l'intérieur du bras creux (52) sur commande manuelle ou robotisée également,
- une prothèse stapédienne (10 ; 10') selon l'une quelconque des revendications 1 à 7, et
- des moyens (58, 60) de fixation des moyens (28) d'actionnement amovibles de la prothèse stapédienne (10 ; 10') au bras creux (52) et à la tige (54) de l'installation.

9. Installation à commande manuelle ou robotisée selon la revendication 8, comportant :
- une prothèse stapédienne (10 ; 10') selon l'une quelconque des revendications 4 à 7,
- des moyens (58, 60) de fixation d'une extrémité proximale du bras creux (38) des moyens d'actionnement amovibles (28) de la prothèse stapédienne (10 ; 10') à une extrémité distale du bras creux (52) déplaçable sur commande manuelle ou robotisée de l'installation, assurant ainsi un positionnement manuel ou robotisé de la prothèse stapédienne (10 ; 10'), et
- des moyens de solidarisation d'une extrémité proximale de la tige coulissante (40 ; 40') des moyens d'actionnement amovibles (28) de la prothèse stapédienne (10 ; 10') à une extrémité distale de la tige (54) déplaçable en translation sur commande manuelle ou robotisée de l'installation, assurant ainsi un rapprochement manuel ou robotisé des deux mâchoires (24, 26 ; 24, 26').

## Patentansprüche

1. Steigbügelprothese (10; 10'), umfassend einen Steigbügelkörper (14), der ein proximales Ende aufweist, versehen mit Mitteln (22) zu Fixierung an der langen Apophyse (12) des Ambosses eines Mittelohrs eines Patienten, und ein distales Ende, das ausgelegt ist, um gegen das Knochenlabyrinth (20) des entsprechenden Innenohrs des Patienten positioniert zu sein, wobei die Fixierungsmittel (22) Folgendes umfassen:
- einen Oberkiefer (24), der derart geformt ist, um auf der langen Apophyse (12) des Ambosses aufliegen zu können,
- einen Unterkiefer (26; 26'), der derart geformt ist, um die lange Apophyse (12) des Ambosses durch Zusammenwirken mit dem Oberkiefer (24) umschließen zu können, und
- Mittel (28) zur Betätigung eines der zwei Kiefer (26, 24; 26', 24) durch Annäherung des anderen der zwei Kiefer (24, 26; 24, 26'), wobei diese Mittel zur Betätigung (28) auf entfernbare Weise an das proximale Ende des Steigbügelkörpers (14) fixiert sind,
**dadurch gekennzeichnet, dass** die entfernbaren Mittel zur Betätigung (28) einen hohlen Arm (38) umfassen, von dem ein distales Ende (38B) auf entfernbare Weise an das proximale Ende des Steigbügelkörpers (14) fixiert sind, und einen Stift (40; 40'), der im Inneren des hohlen Arms (38) gleitet, wobei dieses Gleiten die Annäherung des einen der zwei Kiefer (26, 24; 26', 24) hin zu dem anderen der zwei Kiefer (24, 26; 24, 26') verursacht.

2. Steigbügelprothese (10; 10') nach Anspruch 1, wobei:
- der Unter- (26; 26') und Oberkiefer (24) sich vom proximalen Ende des Steigbügelkörpers (14) erstrecken, um die lange Apophyse (12) des Ambosses umgeben zu können, und
- die entfernbaren Mittel zur Betätigung (28) mit dem Unterkiefer (26; 26') durch nicht elastische Verformung dieses Letzteren zusammenwirken, um es an den Oberkiefer (24) anzunähern.

3. Steigbügelprothese (10; 10') nach Anspruch 2, wobei der Unterkiefer (26; 26') an seinem proximalen Ende (26A; 26A') eine Kerbe (32) zur Unterstützung der Verformung umfasst.

4. Steigbügelprothese (10; 10') nach Anspruch 2 oder 3, wobei die zusätzlichen entfernbaren Mittel zur Betätigung (28) Folgendes umfassen:
- einen hohlen Arm (38), von dem ein distales Ende (38B) auf entfernbare Weise an den Oberkiefer (24) fixiert ist, indem es sich im Wesentlichen in der Verlängerung und gemäß einer Hauptrichtung des Steigbügelkörpers (14) ohne Möglichkeit der Drehung mit Bezug auf diesen Letzteren erstreckt, und
- einen Stift (40; 40'), der im Inneren des hohlen Arms (38) gleitet, von dem ein distales Ende (40B; 40B') auf entfernbare Weise mit dem Unterkiefer (26; 26') zusammenwirkt, um ihn in Annäherung hin zum Oberkiefer (24) durch Gleiten im hohlen Arm (38) zu betätigen.

5. Steigbügelprothese (10; 10') nach Anspruch 4, wobei die entfernbare Fixierung des hohlen Arms (38) an den Oberkiefer (24) mindestens einen zylindrischen Zapfen (42) umfasst, der auf der äußeren Oberfläche des hohlen Arms (38) gebildet ist, der mit mindestens einem Loch (36), das im Oberkiefer (24) gebildet ist, in der Nähe des proximalen Endes des Steigbügelkörpers (14), zusammenwirkt, wobei der mindestens eine zylindrische Zapfen (42) und das mindestens eine Loch (36) in relativer Translation frei in der Richtung ihrer Hauptachsen sind und jede relative Drehung zwischen dem hohlen Arm (38) und dem Oberkiefer (24) um diese Hauptachsen verhindern.

6. Steigbügelprothese (10) nach Anspruch 4 oder 5, wobei das entfernbare Zusammenwirken des gleitenden Stifts (40) mit dem Unterkiefer (26) einen Dorn (30) umfasst, der an einem proximalen Ende (26A) des Unterkiefers (26) gebildet ist, und eine zylindrische Ausbuchtung (40B), die am distalen Ende des gleitenden Stifts (40) gebildet ist, wobei die zylindrische Ausbuchtung (40B) gegen den Dorn (30) anschlägt und die Verformung des Unterkiefers (26) durch Schieben des gleitenden Stifts (40) in eine distale Richtung (A) betätigt.

7. Steigbügelprothese (10') nach Anspruch 4 oder 5, wobei das entfernbare Zusammenwirken des gleitenden Stifts (40') mit dem Unterkiefer (26') eine Ecke (40B') umfasst, die am distalen Ende des gleitenden Stifts (40') gebildet ist, wobei die Ecke (40B') gegen den Unterkiefer (26') anschlägt und seine Verformung durch Zug des gleitenden Stifts (40') in einer proximalen Richtung (B) betätigt.

8. Manuell gesteuerte oder robotergesteuerte Anlage zum chirurgischen Eingriff von Stapedotomie oder Stapedectomie, umfassend:
- einen hohlen Arm (52), der bei manueller Steuerung oder Robotersteuerung verschoben werden kann,
- einen Stift (54), der bei manueller Steuerung oder Robotersteuerung ebenfalls im Inneren des hohlen Arms (52) in Translation verschoben werden kann,
- eine Steigbügelprothese (10; 10') nach einem der Ansprüche 1 bis 7, und
- Mittel (58, 60) zum Fixieren der entfernbaren Mittel (28) zur Betätigung der Steigbügelprothese (10; 10') an den hohlen Arm (52) und an den Stift (54) der Anlage.

9. Manuell gesteuerte oder robotergesteuerte Anlage nach Anspruch 8, umfassend:
- eine Steigbügelprothese (10; 10') nach einem der Ansprüche 4 bis 7,
- Mittel (58, 60) zur Fixierung eines proximalen Endes des hohlen Arms (38) der entfernbaren Mittel zur Betätigung (28) der Steigbügelprothese (10; 10') an ein distales Ende des hohlen Arms (52), das bei manueller Steuerung oder Robotersteuerung der Anlage verschiebbar ist, wobei dadurch eine manuelle oder Roboterpositionierung der Steigbügelprothese (10; 10') sichergestellt wird, und
- Mittel zur festen Verbindung eines proximalen Endes des gleitenden Stifts (40; 40') der entfernbaren Mittel zur Betätigung (28) der Steigbügelprothese (10; 10') an ein distales Ende des Stifts (54), das bei manueller Steuerung oder Robotersteuerung der Anlage verschiebbar ist, wodurch eine manuelle oder Roboterannäherung der zwei Kiefer (24, 26; 24, 26') sichergestellt wird.

## Claims

1. Stapedial prosthesis (10; 10'), comprising a stirrup body (14) having a proximal end provided with means (22) for fixing to the long apophysis (12) of the incus of a middle ear of a patient and a distal end intended to be positioned against the osseous labyrinth (20) of the corresponding inner ear of the patient, the means for fixing (22) comprising:
- an upper jaw (24) designed to be able to bear on the long apophysis (12) of the incus,
- a lower jaw (26; 26') designed to be able to enclose the long apophysis (12) of the incus by cooperation with the upper jaw (24), and
- means (28) for actuating one of the two jaws (26, 24; 26', 24) by bringing it towards the other of the two jaws (24, 26; 24, 26'), these means for actuating (28) being fixed removably to the proximal end of the stirrup body (14),
**characterised in that** the removable means for actuating (28) comprise a hollow arm (38), of which a distal end (38B) is removably fastened to the proximal end of the stirrup body (14), and a rod (40; 40') sliding inside the hollow arm (38), with this sliding causing the bringing of said one of two jaws (26, 24; 26', 24) towards said other of the two jaws (24, 26; 24, 26').

2. Stapedial prosthesis (10; 10') according to claim 1, wherein:
- the lower (26; 26') and upper (24) jaws extend from the proximal end of the stirrup body (14) in such a way as to be able to surround the long apophysis (12) of the incus, and
- the removable means for actuating (28) cooperate with the lower jaw (26; 26') via non-elastic deformation of the latter to bring it towards the upper jaw (24).

3. Stapedial prosthesis (10; 10') according to claim 2, wherein the lower jaw (26; 26') comprises at its proximal end (26A; 26A') a notch (32) for assisting with the deformation.

4. Stapedial prosthesis (10; 10') according to claim 2 or 3, wherein the removable means for actuating (28) comprise:
- a hollow arm (38) of which a distal end (38B) is removably fastened to the upper jaw (24) by extending substantially in the extension and according to a main direction of the stirrup body (14) without possibility of relative rotation with the latter, and
- a rod (40; 40'), sliding inside the hollow arm (38), of which a distal end (40B; 40B') removably cooperates with the lower jaw (26; 26') in order to actuate it by bringing towards the upper jaw (24) by sliding in the hollow arm (38).

5. Stapedial prosthesis (10; 10') according to claim 4, wherein the removable fastening of the hollow arm (38) to the upper jaw (24) comprises at least one cylindrical pin (42), formed on the outer surface of the hollow arm (38), cooperating with at least one hole (36) formed in the upper jaw (24) in the vicinity of the proximal end of the stirrup body (14), said at least one cylindrical pin (42) and said at least one hole (36) being free in relative translation in the direction of their main axes and preventing any relative rotation between the hollow arm (38) and the upper jaw (24) about these main axes.

6. Stapedial prosthesis (10) according to claim 4 or 5, wherein the removable cooperation of the sliding rod (40) with the lower jaw (26) comprises a lug (30) formed at a proximal end (26A) of the lower jaw (26) and a cylindrical bulb (40B) formed at the distal end of the sliding rod (40), the cylindrical bulb (40B) abutting against the lug (30) and actuating the deformation of the lower jaw (26) by pushing the sliding rod (40) in a distal direction (A).

7. Stapedial prosthesis (10') according to claim 4 or 5, wherein the removable cooperation of the sliding rod (40') with the lower jaw (26') comprises a corner (40B') formed at the distal end of the sliding rod (40'), the corner (40B') abutting against the lower jaw (26') and actuating the deformation thereof by traction of the sliding rod (40') in a proximal direction (B).

8. Installation with manual or robotised control of the surgical intervention of stapedotomy or stapedectomy comprising:
- a hollow arm (52) which can be displaced via manual or robotised control,
- a rod (54) that can be displaced in translation inside the hollow arm (52) also via manual or robotised control,
- a stapedial prosthesis (10; 10') according to any of claims 1 to 7, and
- means (58, 60) for fixing the removable means (28) for actuating the stapedial prosthesis (10; 10') to the hollow arm (52) and to the rod (54) of the installation.

9. Installation with manual or robotised control according to claim 8, comprising:
- a stapedial prosthesis (10; 10') according to any of claims 4 to 7,
- means (58, 60) for fixing a proximal end of the hollow arm (38) of the removable means for actuating (28) the stapedial prosthesis (10; 10') to a distal end of the hollow arm (52) which can be displaced via manual or robotised control of the installation, as such providing a manual or robotised positioning of the stapedial prosthesis (10; 10'), and
- means for joining a proximal end of the sliding rod (40; 40') of the removable means for actuating (28) of the stapedial prosthesis (10; 10') with a distal end of the rod (54) that can be displaced in translation on a manual or robotised control of the installation, providing as such manual or robotised bringing together of the two jaws (24, 26; 24, 26').
